(19) Europäisches Patentamt / European Patent Office / Office européen des brevets

(11) **EP 1 770 571 A1**

(12) **EUROPEAN PATENT APPLICATION**

(43) Date of publication:
**04.04.2007 Bulletin 2007/14**

(51) Int Cl.:
***G06F 19/00*** (2006.01)

(21) Application number: **06121164.5**

(22) Date of filing: **25.09.2006**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU**<br><br>(30) Priority: **26.09.2005 FI 20055510**<br>**10.10.2005 FI 20055547** | (71) Applicant: **Medicel Oy**<br>**00290 Helsinki (FI)**<br><br>(72) Inventor: **Varpela, Pertteli**<br>**02730, Espoo (FI)**<br><br>(74) Representative: **Virkkala, Jukka Antero**<br>**Kolster Oy Ab,**<br>**Iso Roobertinkatu 23,**<br>**P.O. Box 148**<br>**00121 Helsinki (FI)** |

(54) **Automatic creation and identification of biochemical pathways**

(57) An information management system for managing biochemical information comprises data structures for modelling biochemical entities (616), categories (606) and biochemical pathways (600). Annotations form many-to-many relationships between biochemical entities and categories. The set of categories comprises function categories for describing a function of each biochemical entity associated to the function category. Interactions (608) model biochemical processes. Each connection (604) associates a biochemical entity (616) or a category (606) to an interaction (608) and has a connection type (610), which either substrate, product, controller and outcome. Each connection has a relation to a pathway (602) and each pathway has a relation to a biochemical location (624). The information management system further comprises an interpretation logic for interpreting each of several categories as a pathway.

Fig. 6

**Description**

**Background of the invention**

The invention relates to an information management system for managing biochemical annotations and pathways, and more particularly to equipment and software products for automatic creation and identification of biochemical annotations and pathways. As used herein, 'biochemical' means biological with or without extensions to chemistry. Biochemical annotations classify biochemical entities to categories. For example, Gene Ontology (GO) Consortium has defined ontologies for annotating gene products to molecular functions, biological processes and cellular components. In addition to the GO system, there are many other category systems, ontologies and controlled vocabularies which are used to annotate biochemical entities to particular categories, in order to describe the functions of the biochemical entities or processes in which they participate. Biochemical pathways are used to model biochemical networks wherein biochemical entities interact with each other.

Biochemical annotations, such as the above-mentioned GO ontology, are based on textual definitions of categories, and they are typically processed manually. Interpretation of such textual definitions of categories requires a biology expert, which may prove out to be a bottleneck in utilizing available information on annotations.

Commonly owned PCT publication WO2005/003999, which is incorporated herein by reference, discloses an exemplary system for modelling specific biochemical systems. While the prior art systems are good at modelling specific biochemical systems as textual categories or individual pathways, they exhibit shortcomings in exploiting similarities and common features between different biochemical systems. There are large amounts of textual information, available both on-line and in printed form, for verbally describing similarities and common features between different biochemical systems but known information systems are incapable of modelling them.

**Brief description of the invention**

An object of the present invention is to provide equipment and software products for modelling biochemical systems such that the above shortcomings are alleviated. The object of the invention is achieved by a equipment and software products which are characterized by what is stated in the independent claims. The preferred embodiments of the invention are disclosed in the dependent claims.

An aspect of the invention is an electronic information management system for managing biochemical information, the information management system comprising data structures for modelling:

- a plurality of biochemical entities;
- a hierarchical structure of a plurality of categories;
- a plurality of pathways;
- a plurality of annotations, wherein each annotation associates a biochemical entity to a category and the plurality of annotations are collectively capable of forming a many-to-many relationship between the plurality of biochemical entities and the plurality of categories;

    - wherein the plurality of categories comprises a plurality of function categories, each function category describing a function of each biochemical entity associated to the function category;

- a plurality of interactions;
- a plurality of connections, wherein each connection:

    - associates a biochemical entity or a category to an interaction and has a connection type, wherein the connection type is selected from a group which comprises substrate, product, controller and outcome, and
    - has a relation to a pathway and each pathway has a relation to a biochemical location; and

- wherein the electronic information management system further comprises an interpretation logic for interpreting each of several categories as a pathway.

This extension of pathway modelling from molecule level to higher component levels, such as cellular compartment, cell, tissue, organ, organism, individual, population, environment, or categories of these entities) makes it possible to utilize automatic molecule-level modelling frameworks, such as those presented in said commonly-owned PCT publication WO2005/003999) where connection information of pathways is used to generate ordinary differential equation models or flux balance models for higher-level biological systems. The above-mentioned data structures support generalizations of biochemical entities and their quantitative variables (eg concentration of cells, tissues, or the like), interactions and their quantitative variables (eg rate of interaction producing cells, tissues) and connections (eg connecting generalized entities to generalized interactions) and their quantitative variables (eg flux via product and substrate connections). This makes it possible to apply similar automatic modelling solutions to all biological systems that are available in prior art systems for chemical or biomolecular systems. To mention just two examples, it will be possible to use flux balance analysis in the study of T-cell maturation process from prethymocytes through some characteristic middle steps to mature thymocytes, or in the steady state of production of epithelial cells when old skin is replaced by new.

A preferred embodiment of the IMS according to the invention further comprises a library of equivalent pathways of categories, wherein each equivalent pathway of a category comprises a set of connections which assigns the set of functions associated to the category to the biochemical entities associated to the category.

Another aspect of the invention is a computer program product, executable in a computer system. The computer program product comprises program code portions for creating the data structures according to claim 1. In other words, the computer program product according to the invention changes a conventional computer system into an IMS according to the invention.

In this IMS description, the references to biochemical entities, interactions or the like should be interpreted as references to data structures which model the biochemical entities, interactions, etc.

An IMS according to the invention is able to treat categories as building blocks of equivalent biochemical pathways.

According to an embodiment of the invention, the IMS further comprises an annotation logic for creating automatic annotations based on the library and specific instances of pathways. For example, the automatic annotations may be created based on pathway topology.

According to another embodiment of the invention, the IMS further comprises an instantiation logic for creating specific instances of pathways based on the library, and an input set of biochemical entities or annotations.

According to yet another embodiment of the invention, the IMS further comprises a generalization logic for creating new categories and/or annotations and/or general pathways based on an input set of specific instances of pathways.

Yet another embodiment of the invention relates to a consistency checker for checking consistency between the annotations, the specific pathways and the library, based on specific instances of pathways and/or general pathways. A benefit of the consistency checker is the ability to automatically check for inconsistencies between the generic and specific pathways and the annotations which define the categories. The annotation logic, instantiation logic, generalization logic and consistency checker may be implemented separately or in combination.

According to a further embodiment of the invention, at least one pathway comprises a hierarchical description of a biochemical entity and a hierarchical description of a location. A benefit of the hierarchical descriptions is the ability to describe biochemical entities and locations with as much detail as is required. The descriptions of biochemical entity and location may be built from a common set of biochemical components but the descriptions are independent from each other, which makes it possible to describe biochemical entities which are located in a non-native location.

Yet another preferred embodiment of the invention comprises means for storing and visualizing descriptions for the biochemical entities and locations in a variable description language ("VDL"). The variable description language comprises variable descriptions, each of which comprises one or more pairs of keyword and name but no line terminator. The pairing of keywords and names makes the VDL largely self-sufficient, or readily processable by computers. An extendible table of permissible keywords supports automatic checking of syntax and/or consistency, yet makes it possible to extend the VDL without programming skills.

**Brief description of the drawings**

In the following the invention will be described in greater detail by means of preferred embodiments with reference to the attached drawings, in which:

Figure 1 is a block diagram of an information management system IMS in which the invention can be used;
Figure 2 illustrates relations between component data, system data and state data;
Figures 3A and 3B show an embodiment of a variable description language (VDL);
Figure 4 illustrates the concept of a hierarchical location information;
Figures 5A and 5B show how annotations associate biochemical entities to categories;
Figure 6 shows how connections couple general categories or specific biochemical entities and interactions to pathways;
Figures 7, 8 and 9A to 9D illustrate an embodiment of an interpretation process;
Figure 10 illustrates the operation of an annotation logic;
Figure 11 illustrates the operation of an instantiation logic;
Figure 12 illustrates the operation of a generalization logic;

Figure 13 illustrates the operation of a consistency checker;
Figure 14 shows a flowchart for an embodiment of the annotation logic;
Figure 15 shows a flowchart for an embodiment of the instantiation logic;
Figure 16 shows a flowchart for an embodiment of the generalization logic;
Figure 17 shows a flowchart for an embodiment of the consistency checker; and
Figures 18A, 18B and 19 to 21 show how the invention can be used to formally express Gene Ontology (GO) definitions.

## Detailed description of the invention

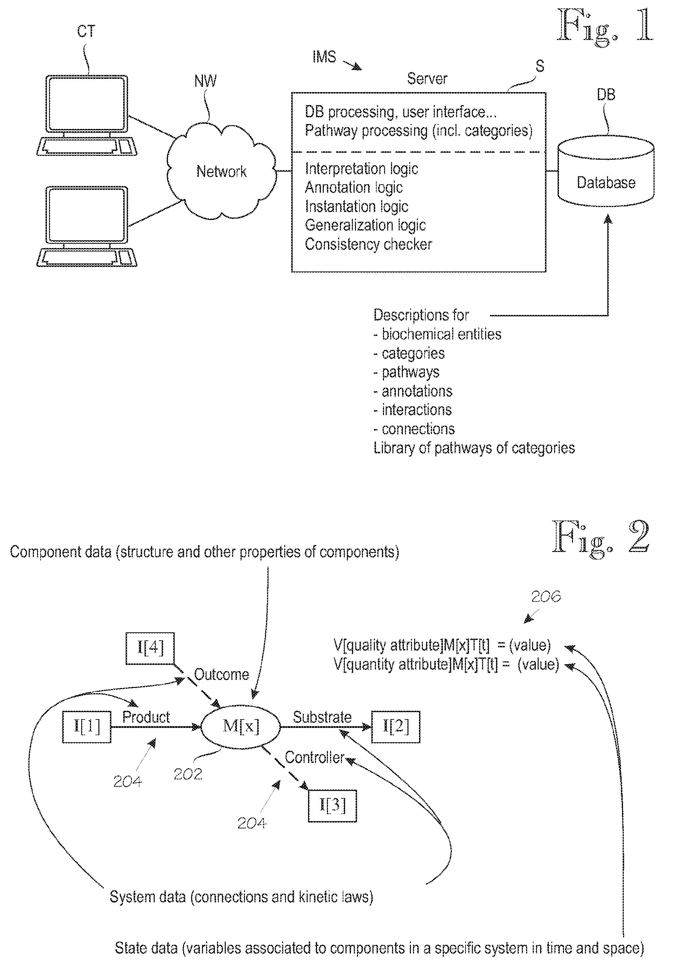

Figure 1 is a simplified block diagram of an information management system IMS in which the invention can be used. In this example, the IMS is implemented as a client/server system but, in principle, the invention is applicable to a single-user system. Several client terminals CT, such as graphical workstations, access a server (or set or servers) S via a network NW, such as a local-area network or the Internet. The server S comprises or is connected to a database DB. The information processing logic within the server and the data within the database constitute the IMS. The database DB is comprised of structure and content. Various preferred embodiments of the invention relate to various processing logics, which are separated from the more common functions of the server by a dashed line.

Figure 2 illustrates relations between different information types. It is beneficial to organize biochemical information into three classes, namely component data, system data and state data.

Components are basic building elements of biochemical systems, such as molecules, cellular compartments, cells (cell types), tissues, organs, organisms, individuals, populations and environments. Component data, which is denoted by reference numeral 202, describes the static properties of components, such as structural or functional features; detected, constant attributes and/or characteristic features. For example, carbon dioxide ($CO_2$) is a component that may have component data. There may also be some variable attributes which do not alter the identity of a biochemical entity.

System data, denoted by reference numeral 204, describes how components are connected to form biochemical systems. The system data 204 also includes the kinetic laws of interaction rates depending on relevant state data, denoted by reference numeral 206. Interactions are transformations in which substrates are converted to products. If a substrate and a product are in different locations, the locations have a common interaction that transports substrates from one location to another as products.

There are connections between interactions and other components. It is advantageous to classify connections into categories which include substrates, products, controllers and outcomes.

In the example shown in Figure 2, there is a substrate type connection between molecule M[x] and interaction I[2]. A substrate type connection means that the biochemical entity or category at the originating end of the connection (here: M[x]) is consumed in the interaction at the terminating end of the connection (here: I[2]).

There is also a product type connection between the molecule M[x] and interaction I[1]. A product type connection means that the biochemical entity or the category at the terminating end of the connection is produced in the interaction at the originating end of the connection.

A controller type connection is a third type of connection, an example of which is the connection from the molecule M[x] to interaction I[3]. A controller type connection means that the biochemical entity or the category at the originating end of the connection (here: M[x]) controls the interaction (eg, its rate) at the terminating end of the connection (here: I[3]).

A fourth type of connection, namely an outcome type connection, means that the biochemical entity or the category at the originating end of the connection (here: M[x]) is modified in terms of attributes in the interaction at the terminating end of the connection (here: I[4]).

A connection may have an associated stoichiometric coefficient to describe kinetic laws (quantitative relations between substrates and products). If the kinetic laws are missing, interaction rates are unknown variables.

Reference numeral 206 collectively denotes state data. There are quantitative and qualitative variables, such as count, concentration, mass, etc., associated to biochemical entities. Quantity attributes are functions of flux rates via product and substrate connections. A representative quantity attribute describes a flux rate of an interaction which transforms a substrate into a product at a certain rate. Quality attributes are functions of outcomes. A representative quality attribute describes the growth of a cell, in which the size of the cell increases by no (new) products are produced. Such variables can be elements of a system's state, which may be described by a set of state data, such as a state vector. State data describes the values of these variables in time and space. For example:

$$V[concentration]U[mol/l]M[CO2]Ts[2005.06.22\ 15:00:00]L[my\_location] = 1.5$$

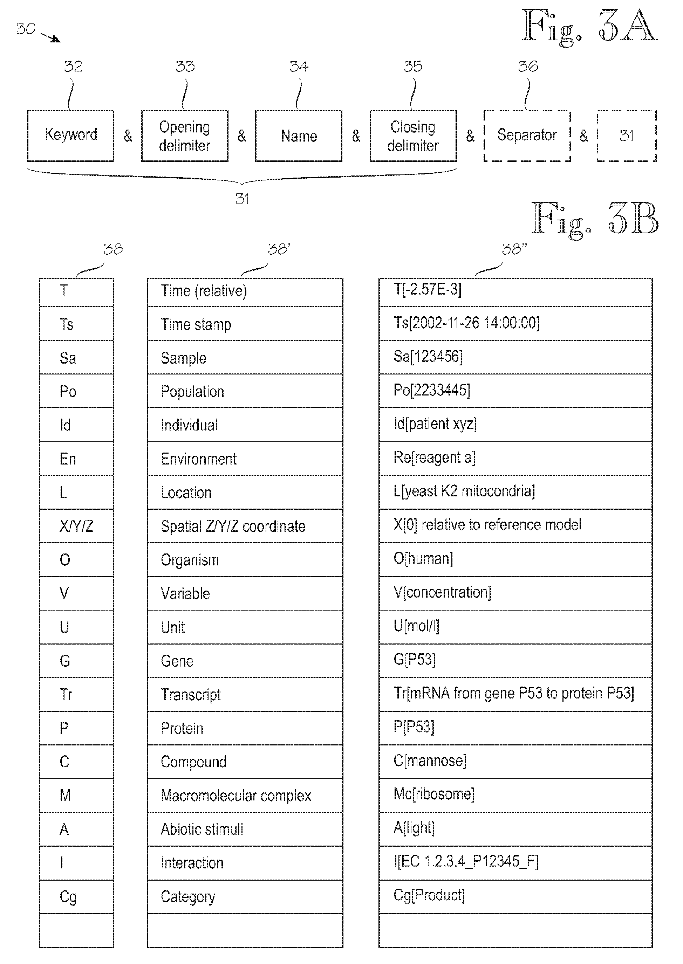

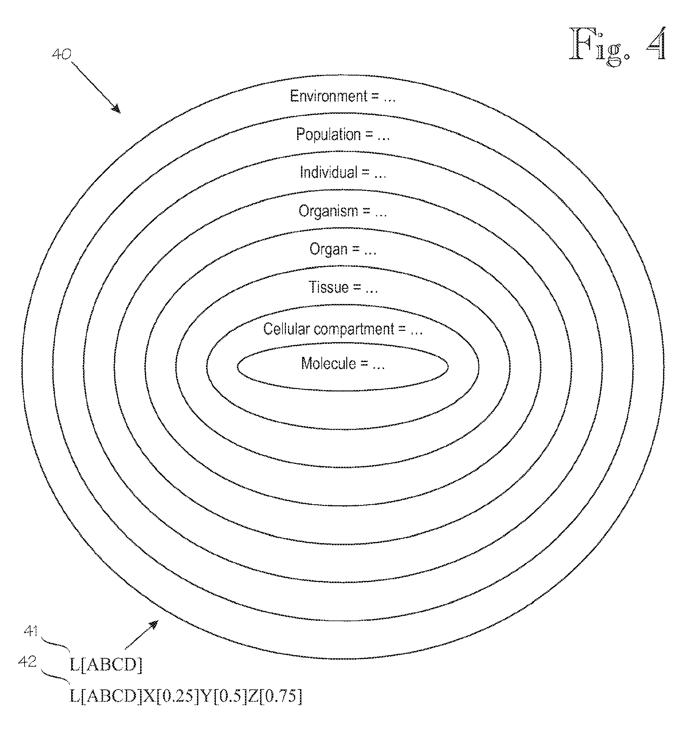

This is an expression of a variable (concentration) expressed in units (mol/l) of molecule $CO_2$ at time stamp 22 June 2005 at 15:00 in a location called "my_location". The value of the variable is 1.5. Such variables are preferably expressed in a systematic variable description language (VDL), which will be further described in connection with Figures 3A and 3B. Location information will be further described in connection with Figure 4.

Space can be a discrete location, eg "my_location", which may be specified in terms of an environment, population, individual, organism, organ, tissue, cell type, or cellular compartment. Some of these location-specifying elements may be not applicable or be used to specify the location. In addition to specifying location information based on biochemical elements, the location information can be specified spatially, by using a reference coordinate system. For example:

$$V[concentration]U[mol/l]M[CO2]Ts[2005.06.22\ 15:00:00]L[my\_location]X[0.5]Y[0.2]Z[0.5] = 1.5$$

Figures 3A and 3B show an embodiment of a variable description language (VDL). Generally speaking, a variable is anything that has a value and represents the state of a biochemical system (either a real-life biomaterial or a theoretical model). When an IMS is taken into use, the designer does not know what kinds of biomaterials will be encountered or what kinds of experiments will be carried out or what results are obtained from those experiments. Accordingly, variable descriptions have to be open to future extensions. On the other hand, openness and flexibility should not result in anarchy, which is why well-defined rules should be enforced on the variable descriptions. These needs are best served by an extendible variable description language ("VDL").

eXtendible markup language (XML) is one example of an extendible language that could, in principle, be used to describe biochemical variables. XML expressions are rather easily interpretable by computers. However, XML expressions tend to be very long, which makes them poorly readable to humans. Accordingly, there is a need for an extendible VDL that is more compact and more easily readable to humans and computers than XML is.

The idea of an extendible VDL is that the allowable variable expressions are "free but not chaotic". To put this idea more formally, we can say that the IMS should only permit predetermined variables but the set of predetermined variables should be extendible without programming skills. For example, if a syntax check to be performed on the variable expressions is firmly coded in a syntax check routine, any new variable expression requires reprogramming. An optimal compromise between rigid order and chaos can be implemented by storing permissible variable keywords in a data structure, such as a data table or file, that is modifiable without programming. Normal access grant techniques can be employed to determine which users are authorized to add new permissible variable keywords.

Figure 3A illustrates a variable description in a preferred VDL. A variable description 30 comprises one or more pairs 31 of a keyword and name, separated by delimiters. As shown in the example of Figure 3A, each keyword-name pair 31 consists of a keyword 32, an opening delimiter (such as an opening bracket) 33, a (variable) name 34 and a closing delimiter (such as a closing bracket) 35. For example, "Ts[2002-11-26 18:00:00]" (without the quotes) is an example of a time stamp. If there are multiple keyword-name pairs 31, the pairs can be separated by a separator 36, such as a space character or a suitable preposition. The separator and the second keyword-name pair 31 are drawn with dashed lines because they are optional. The ampersands between the elements 32 to 36 denote string concatenation. That is, the ampersands are not included in a variable description.

As regards the syntax of the language, a variable description may comprise an arbitrary number of keyword-name pairs 31. But an arbitrary combination of pairs 31, such as a concentration of time, may not be semantically meaningful.

Figure 3B shows a table 38 of typical keywords. Next to each entry in table 38 is its plaintext description 38' and an illustrative example 38". Note that the table 38 is stored in the IMS but the remaining tables 38' and 38" are not necessarily stored (they are only intended to clarify the meaning of each keyword in table 38). For example the example for keyword "T" is "T[-2.57E-3]" which is one way of expressing minus 2.57 milliseconds prior to a time reference. The time reference may be indicated by a timestamp keyword "Ts".

The T and Ts keywords implement the relative (stopwatch) time and absolute (calendar) time, respectively. A slight disadvantage of expressing time as a combination of relative and absolute time is that each point of time has a theoretically infinite set of equivalent expressions. For example, "Ts[2002-11-26 18:00:30]" and "Ts[2002-11-26 18:00:00]T[00:00:30]" are equivalent. Accordingly, there is preferably a search logic that processes the expressions of time in a meaningful manner.

By storing an entry for each permissible keyword in the table 38 within the IMS, it is possible to force an automatic syntax check on variables to be entered, as shown in Figure 3C of said PCT publication W02005/003999.

The syntax of the preferred VDL may be formally expressed as follows:
<variable description>::=<keyword>"["<name>"]"{{separator}<keyword>"["<name>"]"}<end>
<keyword>::=<one of predetermined keywords, see eg table 38>
<name>::=<character string> |"*" for any name in a relevant data table

The purpose of explicit delimiters, such as "[" and "]" around the name is to permit any characters within the name, including spaces (but excluding the delimiters, of course).

A preferred set of keywords 38 comprises three kinds of keywords: what, where and when. The "what" keywords, such as variable, unit, biochemical entity, interaction, etc., indicate what was or will be observed. The "where" keywords, such as sample, population, individual, location, etc., indicate where the observation was or will be made. The "when" keywords, such as time or time stamp, indicate the time of the observation. The "what", "where" and "when" keywords are separate and independent of one another, which makes it possible to describe the location of a biochemical entity independently of its function, for example.

In the set of permissible keywords 38 shown in Figure 3B, "M" stands for macromolecular complex, but elsewhere in this description, VDL expressions like "M[xyz]" serve as examples of any biochemical entity.

A key feature of the VDL described in connection with figures 3A and 3B is the lack of line termination characters (new line, carriage return, or the like). This feature helps achieve very compact VDL expressions, unlike the expressions in XML and its derivatives which are very verbose. However, the VDL described herein shares a principal benefit of XML, namely self-sufficiency, which means that little or no external information (apart from the syntax of the VDL and the list of permissible keywords) is required to interpret the VDL expressions.

Figure 4 illustrates the concept of a hierarchical location information, in which the location of a sample of biomaterial or pathway is expressed as a hierarchy of component data. Location serves as a concept that helps to specify where the biochemical entities are located, where they interact (pathways are related to specific locations), and/or where biomaterial samples are obtained, for quantifying the biochemical entities and so on. Location data can be used to relate different data properly between different hierarchy levels. Properly identified instances of locations can be treated as discrete locations. In spatial considerations all discrete locations can be used as references where locations can be spatially specified by relative co-ordinates to discrete reference locations.

Reference numeral 40 denotes a set of components for describing a hierarchical location. The outmost component of the set of components 40 is called an environment. The environment may be the natural environment of sample population or an individual, or it may determine the conditions of experiments. Environment can be registered as a component of a location. In general, the description of an environment may contain all the component classes smaller than the environment, such as populations, individuals, organisms, organs, tissues, cells, cellular compartments and molecules. If relevant, there can be progressively smaller location components hierarchically inside others.

A description of a location can be modelled to hold any set of relevant components from the following hierarchical levels of location: environment, population, individual, organism, organ, tissue, cell type and cellular compartment. Molecule classes are the most basic components that can be located in all upper level discrete locations. These levels correspond to main classes of biochemical entities. There may be hierarchical categories of biochemical entities at each main level of components. Each location instance specifies relevant instances of relevant hierarchical levels. Reference numeral 41 denotes an instance of a hierarchical location which is expressed in terms of the set of components 40. Reference numeral 42 is an even more specific location instance which further defines the location 41 by a three-dimensional coordinate system {X, Y, Z}.

Each location instance specifies relevant instances of relevant hierarchical levels. Comparability of different locations is supported by standardized main levels of location concept and available ontologies at least for some of the levels.

The hierarchical location information provides certain advantages. For example, a location information may be arbitrarily specific, down to spatial coordinates within a cell, yet searchable by queries which express the location in any hierarchical level, such as "heart", "human" or "human heart". In other words, the hierarchical location information can be seen as a mechanism for zooming in and out within the component structures. Component data, system data and state data can be applied at all different levels of systems

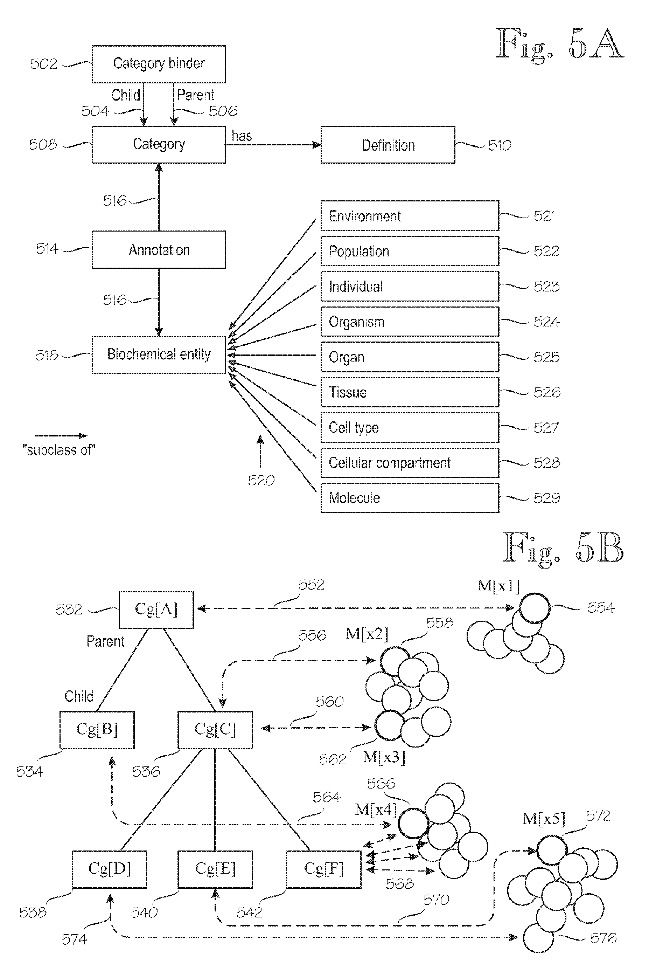

Figures 5A and 5B show how annotations associate biochemical entities to categories. An element of the invention is a hierarchical structure of categories. The structure of categories comprises a plurality of function categories, wherein each function category describes one or more functions of each biochemical entity associated to the function category.

In addition to the function categories, there may be location categories and/or process categories. Location categories indicate where the entities associated with the category are located in or what they are part of. Process categories indicate processes in which the entities associated with the category participate in.

In the embodiment shown in Figure 5A, the hierarchical structure of categories is implemented by means of category binders, denoted by reference numeral 502. A category binder may have a child relation 504 or a parent relation 506 to a category 508. This means that one category can be a child of another category and a parent of yet another category, whereby the category binders 502 connect the categories 508 in a truly hierarchical structure. Each category 508 has a definition 510.

There is also a set of annotations 514. Each annotation has association relations, denoted by reference numeral 516, between a biochemical entity 518 and a category. Each biochemical entity 518 can be described by a hierarchy 520 of specifiers 521 - 529, whereby the biochemical entities can be described at any desired level of detail. For example, if the specifiers organism 524 and organ 525 are present, the biochemical entity can be a human heart or a feline eye. But further specifiers can be added to the hierarchy 520 to describe the biochemical entity in terms of a specific environment 521, population 522 or individual 523, or down to a detail level of a specific molecule 529.

The set of annotations 514 are collectively capable of forming a many-to-many relationship between the set of biochemical entities 518 and the set of categories 508. Such many-to-many relationship are shown in Figure 5B in which the solid lines denote child-parent relations between the categories Cg[A] to Cg[F], and the dashed lines denote associations between biochemical entities (here: molecules M[1] to M[5]) and the categories. For example category Cg[A] 532 is a parent of categories Cg[B] 534 and Cg[C] 536, of which the latter is a parent of categories Cg[D] 538, Cg[E] 540 and Cg[F] 542. Association 552 joins element 554 of molecule M[x1] to category Cg[A] 532. Associations 556 and 560 join element 558 of molecule M[x2] and element 562 of molecule M[x3] to category Cg[C] 536. Association 564 joins element 566 of molecule M[x4] to category Cg[B] 534, while some related elements are joined to Cg[F] 542 by associations 568, etc.

The data structure shown in Figures 5A and 5B improves the usability and availability of biochemical information. The controlled vocabularies and ontologies of the prior art systems provide free-format verbal descriptions of biochemical systems but they lack the formalism of the present invention which is necessary to make such description understandable to present computers.

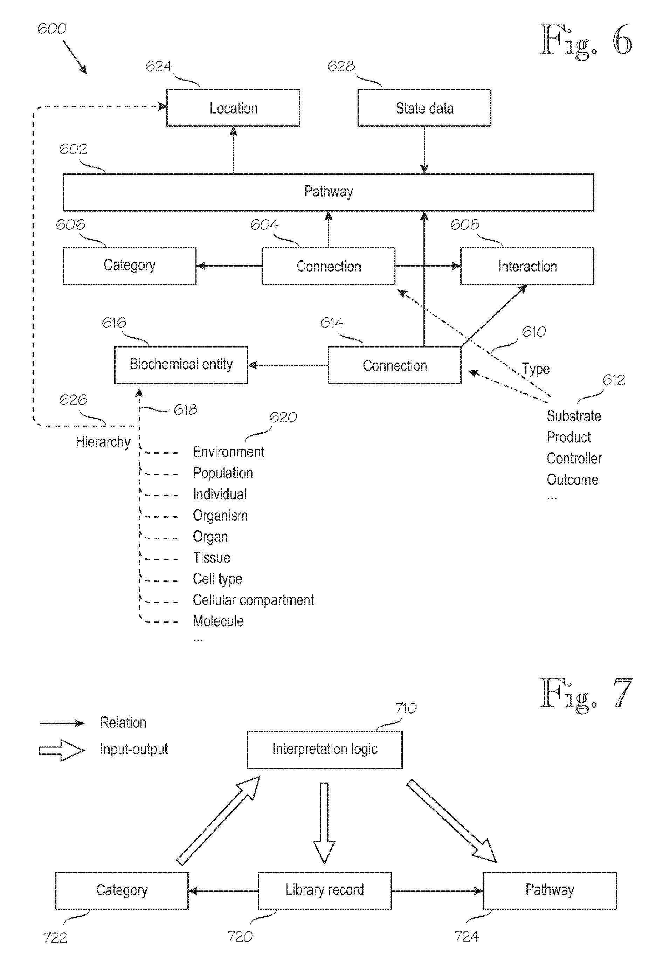

Figure 6 shows how connections couple general categories or specific biochemical entities and interactions to pathways. Figure 6 is an entity-relationship model of a preferred data structure for modelling biochemical pathways. The data structure shown in Figure 6 comprises several distinctive features. First, there is a separate connection data element 614 that connects a biochemical entity 616 and an interaction 608, as opposed to a data structure in which, say, each data element for a biochemical entity 616 has a "to" information field which points directly to the interaction 608, ie, without the separate connection element 614. A benefit of a separate connection element 614 is the ability to maintain proper many-to-many relations within the pathways.

Second, each connection 604 has an associated type element 610. The set of type values indicates the type of the connection. The set of type elements 612 includes at least substrate, product, outcome and controller. These types were previously described in connection with Figure 2.

Third, the biochemical entities 616 are described as hierarchies 618 which are composed of components, collectively denoted by reference numeral 620. A benefit of the hierarchical description of biochemical entities is the ability to describe the validity of pathways at any level of detail. For example, some pathways may be valid for any animals, while some are valid for only a specific organ or a specific individual.

Fourth, the pathway 602 has a relation to a specific location information 624. A location 624, which is separate from the biochemical entity 616, makes it possible to describe biochemical systems in which a biochemical entity is transferred to a location different from its native or original location. The location information may also comprise a hierarchy 626 composed of the components 620. But although the biochemical entity description 616 and the location hierarchy 626 are both hierarchical description composed of the components 620, they are separate information structures, whereby the pathway shown in Figure 6 is fully capable of modelling scenarios in which a specific allergen (an example of a hierarchical biochemical entity 616) is in a non-native location 624, such as in a different organism, which is a specific instance of a location hierarchy 626.

Finally, not only biochemical entities 616 but also categories 606 are connected to interactions 608 by connection data elements 604. A benefit of this feature is that the pathways can be more generic. For example, this feature saves memory. If each of a number N molecules is capable of acting as a biochemical entity 616 in a pathway 602, there is no need to store N separate pathways. Instead, each of the N molecules is associated to a category 606, which is then used as a building block in the pathway 602.

In addition to the above-described data elements, the data structure 600 describing a pathway may also include state data, which is collectively denoted by reference numeral 628. State data was previously described in connection with Figure 2.

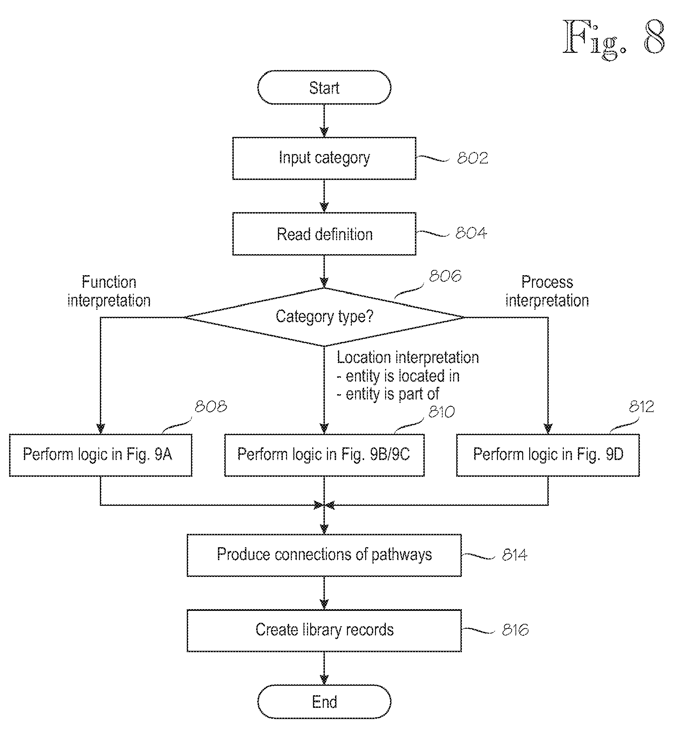

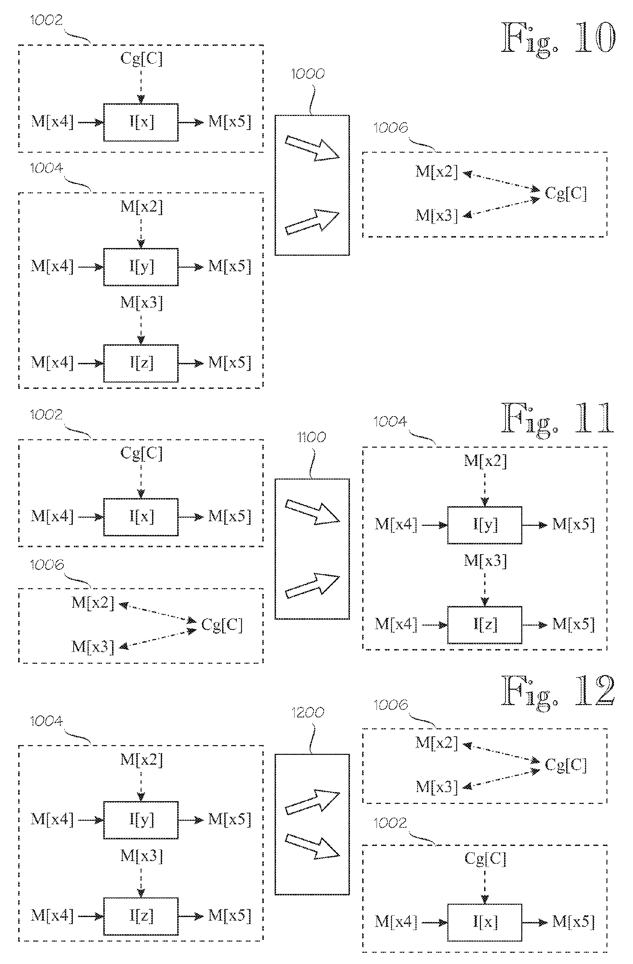

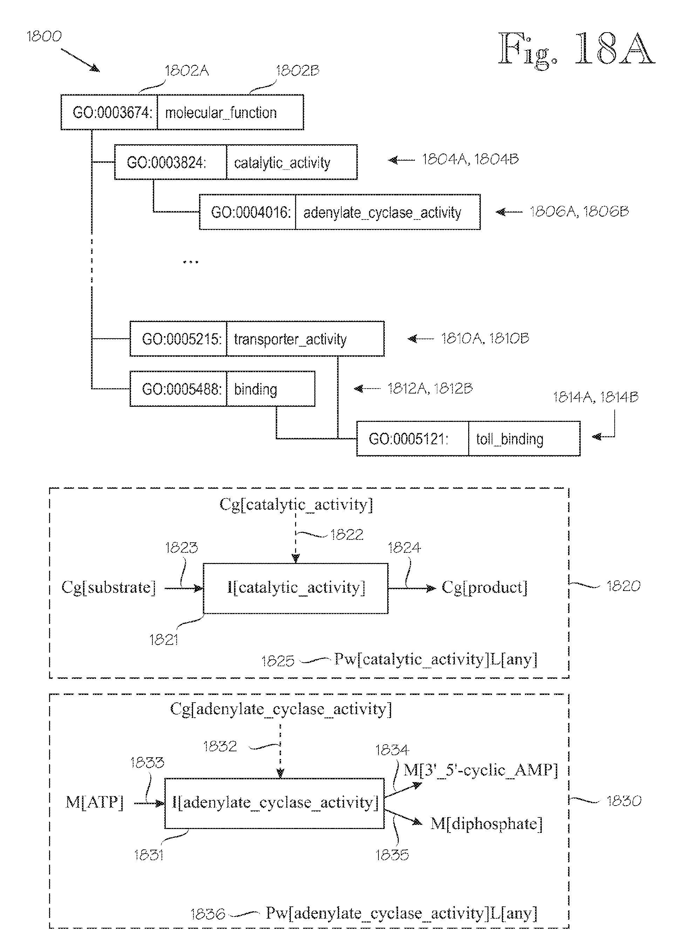

Figures 7, 8 and 9A to 9D illustrate an embodiment of an interpretation logic 710. The purpose of the interpretation logic 710 is to represent the biochemical meanings of the categories by equivalent pathways if possible. The process is somewhat analogous to replacing complicated electro-physical phenomena by equivalent circuits. An example will be shown in Figure 6. In other words, the interpretation logic 710 aims at replacing the category definitions by the set of connections, wherever possible. The input of the interpretation logic 710 is the set of categories 722, and, indirectly, the category definitions (item 510 in Figure 5). its output includes the set of connections of a pathway, denoted by reference numeral 724 and a set of library records 720 which associate each category 720 with a pathway 724. Automation of the interpretation logic 710 is not critical because the number of useful categories is small compared with the number of annotations of the biochemical entities to the categories. Accordingly, the interpretation logic 710 may be implemented as a logic which displays the categories and their definitions to a human expert and records the response of the expert in a database. But even in such a rudimentary interpretation logic, the expert's responses have to be entered only once and they are available at any time to all users of the information management system as systematic pathway models which are understandable to humans when visualized and processable by computers as database records. Thus if there is a library of equivalent pathways of categories, regardless of how the library has been created, the free-format verbal descriptions can be replaced by relevant structures of connection data which can be used systematically in several different applications of data processing. Further examples will be shown in connection with Figures 10 - 17. Examples of categories and equivalent pathways will be shown in connection with Figures 18A - 21.

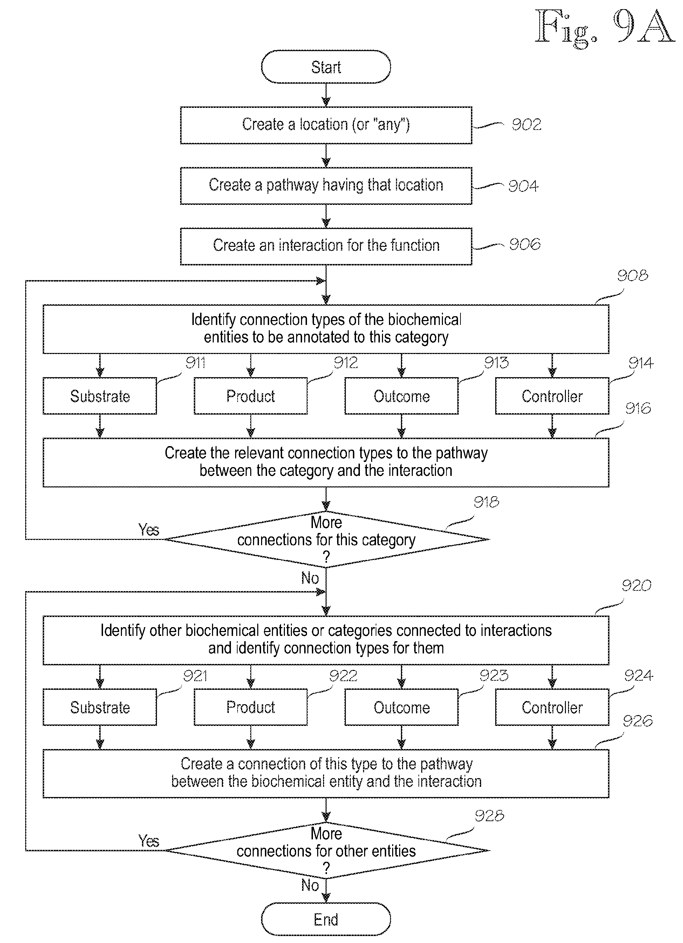

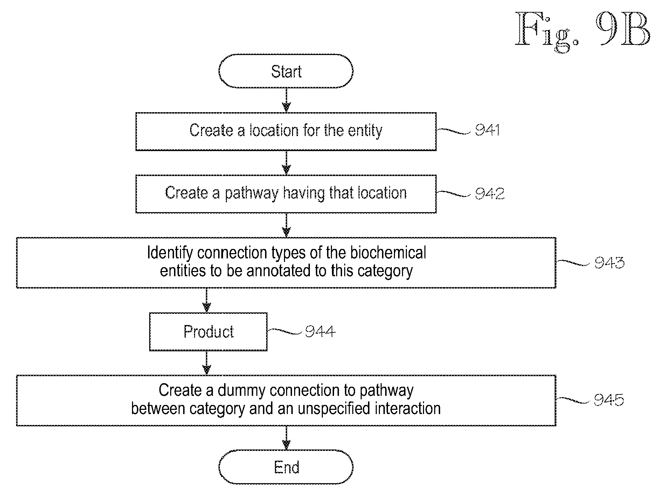

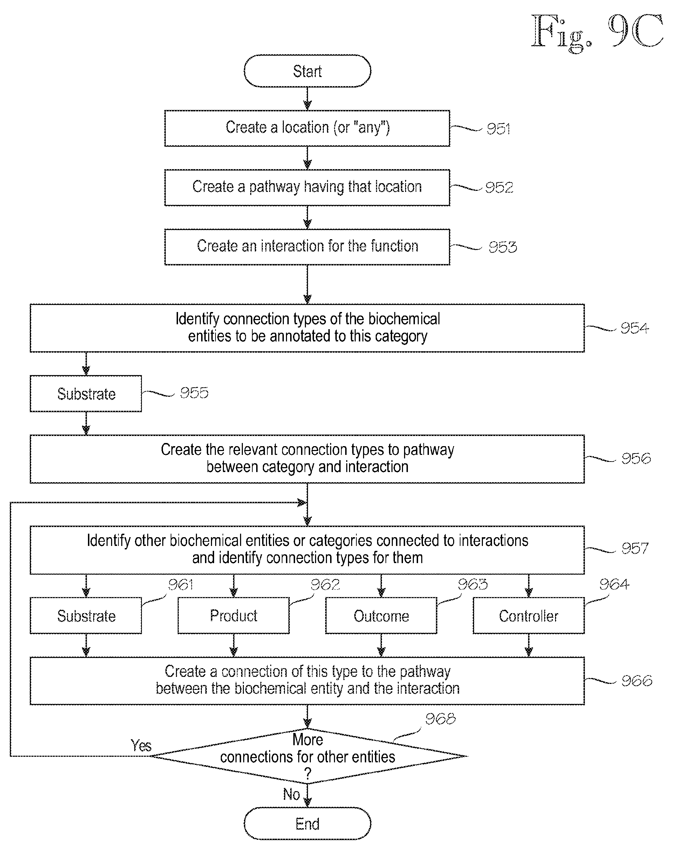

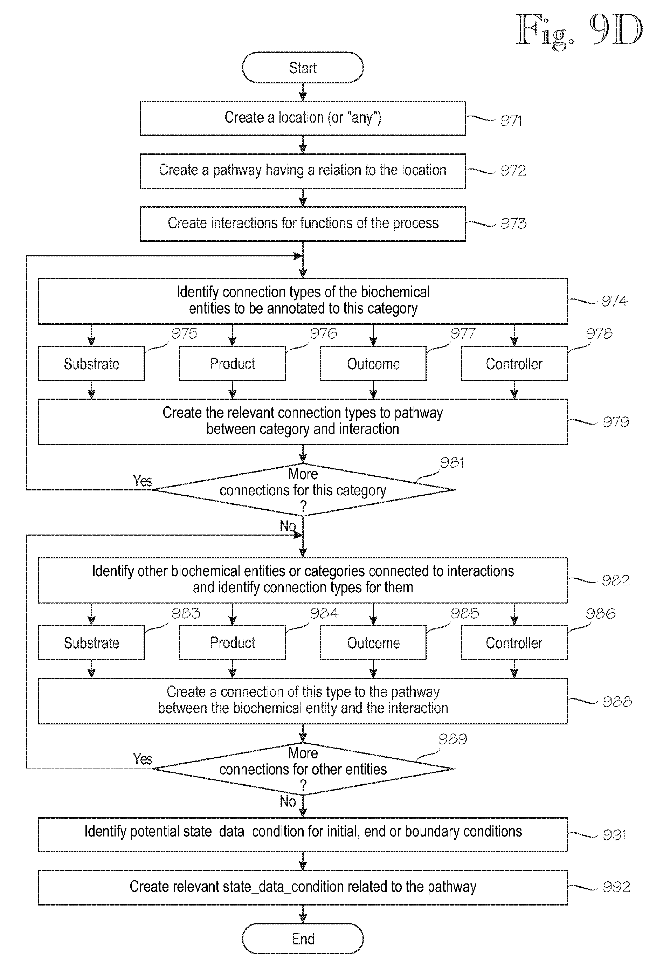

For special high-volume cases, the interpretation logic may be automated. Figures 8 and 9A to 9D illustrate flowcharts for an interpretation logic. In step 802 of Figure 8, the interpretation logic inputs a category identifier. The category identifier may be entered by a human user or another software application. In step 804 the interpretation logic reads the category definition from a database (see item 510 in Figure 5A). In step 806 the interpretation logic determines the type of the category. If the type is a function interpretation, step 808 is performed, which step is shown in more detail in Figure 9A. If the type of the category is location interpretation, step 810 is performed. There are two types of location interpretation. A first type concerns where a biochemical entity is located in. Figure 9B shows the steps for this process. A second type concerns what a biochemical entity is part of. Figure 9C shows the steps for this process. Finally, if the type of the category is process interpretation, step 812 is performed, which step is shown in more detail in Figure 9D.

In step 814 the interpretation logic produces the connections of pathways. In step 816 it creates the relevant library records.

Figure 9A shows the steps performed by an interpretation logic when performing a function interpretation. In step 902 the interpretation logic creates a relevant location. An "undefined" location, in which all hierarchical location components are "undefined", can be used to indicate a definition which does not specify a location. In step 904 the interpretation logic creates (initializes) a pathway having a relation to that location. In step 906 the interpretation logic creates an interaction for the function.

In step 908 the interpretation logic identifies the connection types of the biochemical entities which are to annotated to the present category. In steps 911, 912, 913 and 914, the connection type is respectively prepared as substrate, product, outcome or controller. In step 916 the interpretation logic complements the pathway with relevant connections and types between the category and the interaction (see item 612 in Figure 6). The test in step 918 causes a return to step 908 if there are more connections for the present category. If the connections have been exhausted, the logic executes step 920 in which it identifies other biochemical entities or categories which are connected to the interactions. The logic also determines appropriate connection types for such biochemical entities or categories. In steps 921, 922, 923 and 924, the connection type is identified as substrate, product, outcome or controller, respectively. In step 926 a connection of the identified type is created in the pathway between the biochemical entity and the interaction. The test in step 928 causes a return to step 920 if there are more connections for other entities. Otherwise the logic shown in Figure 9A is completed and the process continues to step 814 shown in Figure 8.

Figure 9B shows the steps relevant to the case in which the interpretation logic determines a location where a biochemical entity is located in. Steps 941, 942 and 943 correspond to steps 902, 904 and 906, respectively, and will not be described again. In step 944 the biochemical entity is identified as a product. In step 945 the interpretation logic creates a dummy connection to the pathway between the category associated to the biochemical entity and an unspecified interaction. An example will be shown in connection with Figure 20. Then the process continues to step 814 shown in Figure 8.

Figure 9C shows the steps relevant to the case in which the interpretation logic determines a location which a biochemical entity is a part of. Steps 951, 952 and 954 correspond to steps 902, 904 and 906, respectively, and will not be described again, but step 954 is preceded by step 953 in which the interpretation logic creates an interaction for the function in question. In step 955 the biochemical entity is identified as a substrate. In step 956 the interpretation logic creates the relevant connection types to the present pathway between the category associated to the biochemical entity and the interaction created in step 953. From this point on, the process in Figure 9C is similar to the one shown in Figure 9A, steps 920 - 926, and the description will not be repeated.

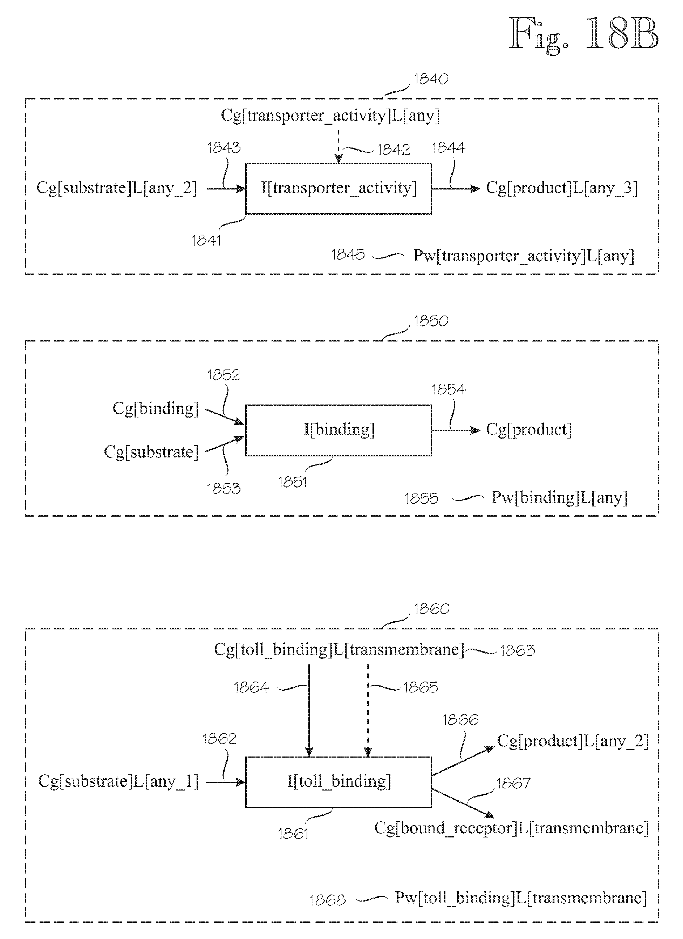

Figure 9D shows the steps executed in process interpretation. Most of the steps, up to and including step 989, have corresponding steps in Figures 9A to 9C, and a repeated description is omitted. In step 991 the interpretation logic identifies potential state data conditions for the initial and end states and any applicable boundary conditions. In step 992 the interpretation logic creates the relevant state data conditions related to the pathway. An example will be shown in connection with Figure 19.

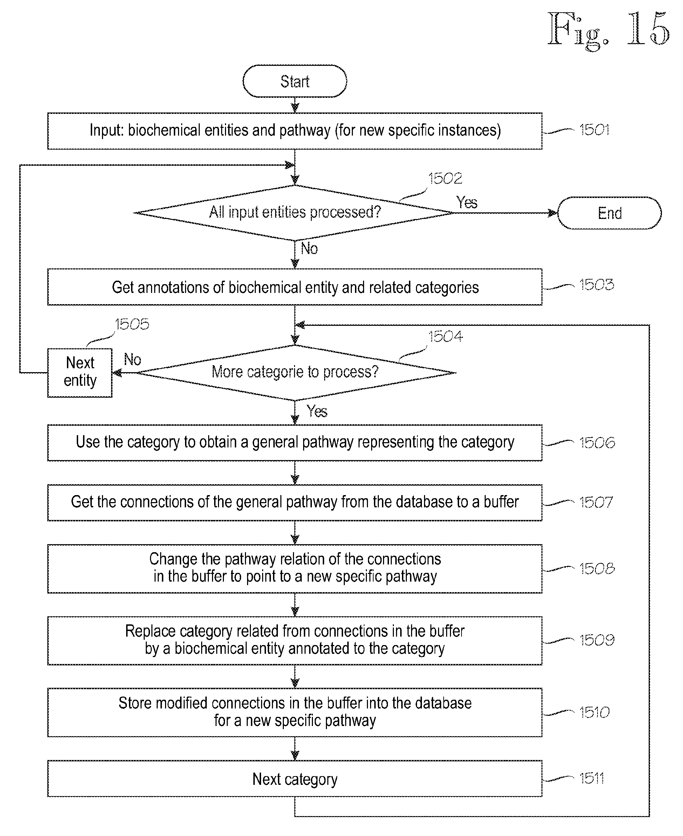

Figures 10 to 13 illustrate the operation of various automation logics, namely annotation logic, instantiation logic, generalization logic and a consistency checker, when these logics are seen as "black boxes". Flowcharts for implementing exemplary embodiments of these logics will be described later, in connection with Figures 14 to 17.

Figure 10 illustrates the operation of an annotation logic 1000. The annotation logic automatically creates annotations that associate given biochemical entities to categories. The annotation logic 1000 has two inputs, namely a general pathway 1002 and a set 1004 of specific pathways. The general pathway 1002 indicates that any biochemical entity in category Cg[C] acts as a controller in an interaction I[x] which transforms molecule M[x4] to molecule M[x5].

The set 1004 of specific pathways indicates that molecules M[x2] and M[x3] are both capable of acting as controllers in interactions I[y], I[z] which transform molecule M[x4] to molecule M[x5]. In other words, the molecules M[x2] and M[x3] both fulfil the definition for the category Cg[C]. Based on this information, the annotation logic 1000 is capable of creating a set 1006 of annotations which annotate molecules M[x2] and M[x3] to category Cg[C].

Figure 11 illustrates the operation of an instantiation logic 1100. The instantiation logic 1100 creates specific instances of general pathways. The instantiation logic 1100 operates on the same data sets as the annotation logic 1000 but the roles of the specific pathways 1004 and the annotations 1006 are reversed. The instantiation logic 1100 has two inputs, namely the general pathway 1002 and the set 1006 of annotations. Based on the inputs, the instantiation logic 1100 is capable of creating the set 1004 of specific pathways.

Figure 12 illustrates the operation of a generalization logic 1200. It has only one input, namely the set 1004 of specific pathways. The generalization logic 1200 detects the similarities between the two pathways, the only difference being the molecule (M[x2] or M[x3]) acting as a controller in the interactions I[y] and I[z]. Based on the similarity of the pathways, the generalization logic 1200 first detects that it is useful to create category Cg[C] and creates the set 1006 of annotations which annotate molecules M[x2] and M[x3] to the category. The generalization logic 1200 then generalizes the set 1004 of specific pathways by creating the general pathway definition 1002 in which the category Cg[C] is substituted for the specific molecules M[x2] and M[x3].

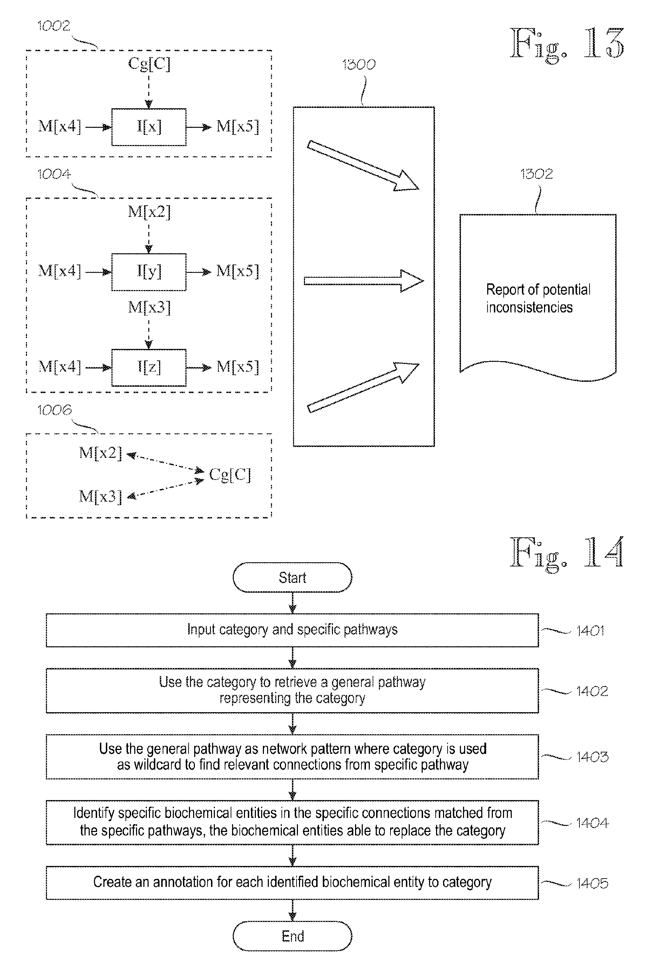

While each of the annotation logic 1000, instantiation logic 1100 and generalization logic 1200 are usable on their own, a combination of all these three logics is particularly advantageous. In addition to these three logics, an advantageous embodiment of an information management system also comprises a consistency checker 1300, an embodiment of which is shown in Figure 13. The inputs to consistency checker 1300 comprise a general pathway definition 1002, a set 1004 of specific pathway definitions and a set 1006 of annotations. The consistency checker 1300 checks if the information in the input data sets is consistent and creates a report 1302 of potential inconsistencies.

It should be understood that Figures 10 to 13 are simplified and only serve to illustrate the operation of these logics. In real-life situations, the general and specific pathways are typically much more complex than the simplified drawings shown in Figures 10 to 13. They also contain a far greater number of connections of various types which connect virtually any kinds of biochemical entities to any interactions. In addition to the inputs shown, the logics typically have a user interface via which a user may specify what operations to perform, what the input data set is, and so on.

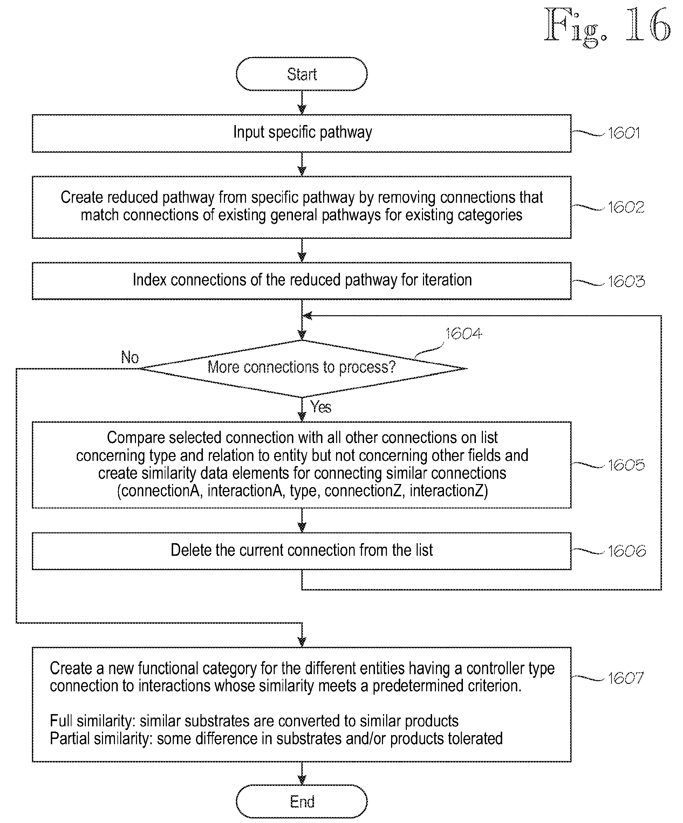

Figure 14 shows a flowchart for an embodiment of the annotation logic. The overall operation of the annotation logic was discussed in connection with Figure 10. In step 1401 the annotation logic receives a category identifier and a set of specific pathways from a user interface or another software application. In step 1402 the annotation logic uses the received category identifier to obtain a definition of a general pathway which matches the category (see item 1002 in Figure 10). In step 1403 it uses the general pathway as a network pattern, such that the interaction and the category are used as wildcards to find relevant connections from each of the specific pathways (such as items 1004 in Figure 10). A pattern-matching logic suitable for this purpose has been described in commonly-owned European Patent Application EP 1 494159 A (or US patent application 10/883,648), particularly in connection with Figures 16A to 16E.

In step 1404 the annotation logic identifies specific biochemical entities that appear to be valid replacements for the category. In step 1405 the annotation logic creates an annotation to the category for each identified biochemical entity (see item 510 in Figure 5A and item 1006 in Figure 10).

Figure 15 shows a flowchart for an embodiment of the instantiation logic. The overall operation of the instantiation logic was discussed in connection with Figure 11. In step 1501 the instantiation logic receives an input from a user interface or another software application. The input indicates a set of biochemical entities. The input also indicates a pathway identifier which will identify an existing pathway which is to be completed or an entirely new pathway. In step 1502 the logic checks if all inputted entities have been processed. If yes, the process ends. If not, the logic proceeds to step 1503 for obtaining the annotations of the current biochemical entity and its related categories. In step 1504 the logic checks if the current biochemical entity has more related categories to process. If not, the logic proceeds to step 1505 for processing the next biochemical entity and returns to step 1502. Otherwise the logic proceeds to step 1506 in which the logic uses the description of the current category to obtain a general pathway which represents the current category. In step 1507 the logic retrieves the connections of the general pathway from the database to a temporary buffer. In step 1508 the logic modifies the connections in the buffer such that pathway relation of the connections points to a new specific pathway. In step 1509 the logic replaces the category which has relations from the connections in the buffer by a biochemical entity which is annotated to the category. In step 1510 the logic stores the modified connections in the buffer into the database as a new specific pathway. In step 1511 the logic obtains the next category and returns to step 1504.

Figure 16 shows a flowchart of an embodiment of the generalization logic. In step 1601 the generalization logic receives an input which indicates a set of specific pathways. In step 1602 the logic creates a reduced pathway from the set of specific pathways by removing connections which match connections of existing general pathways for existing categories. The aim is thus to prevent creation of redundant categories. In step 1603 the logic indexes the connections of the reduced pathways. In other words, the logic creates an indexed list of the connections, in order to be able to process each of the connections. In step 1604 the logic checks if there are unprocessed connections. If yes, the process continues to step 1605 in which the logic compares a selected connection with all other connections in the list, wherein the comparison comprises comparing the types and relations to the biochemical entity, while ignoring other fields, and creates similarity descriptors (data structures describing similarity) for connecting similar connections. In step 1606 the current (=already processed) connection is deleted from the indexed list and the process returns to step 1604.

When all connections have been processed the logic proceeds to step 1607 in which the logic creates a new functional category for the different entities having a controller type connection to interactions whose similarity meets a predetermined criterion. The new pathway in the new functional category is a generalization of the interactions and connections having similarity descriptors. For example, in case of Figure 12, the specific pathway 1004 has two similarity descriptors. One similarity descriptor is formed by connection M[x4]I[y], interaction I[y], substrate connection M[x4]I[z] and interaction I[z]. The other similarity descriptor is formed by connection M[x5]I[y], interaction I[y], product connection M[x5]I[z] and interaction I[z]. The similarity descriptors make it possible to generalize the interactions I[y] and I[z] to I[x] and substrate connections M[x4]I[y] and M[x4]I[z] to substrate connection M[x4]I[x] and product connections M[x5]I[y] and M[x5]I[z] to product connection M[x5]I[x] for a new general pathway of a new category Cg[C]. The new category Cg [C] acts as a controller to interaction I[x] the same way as biochemical entities M[x2] and M[x3] acts as controllers to interactions I[y] and I[z] respectively.

If full similarity is required, the new functional category is created only for interactions in which similar substrates are converted to similar products. If partial similarity is sufficient, the new functional category is created for interactions in which the combination of substrates and products differs in some respects.

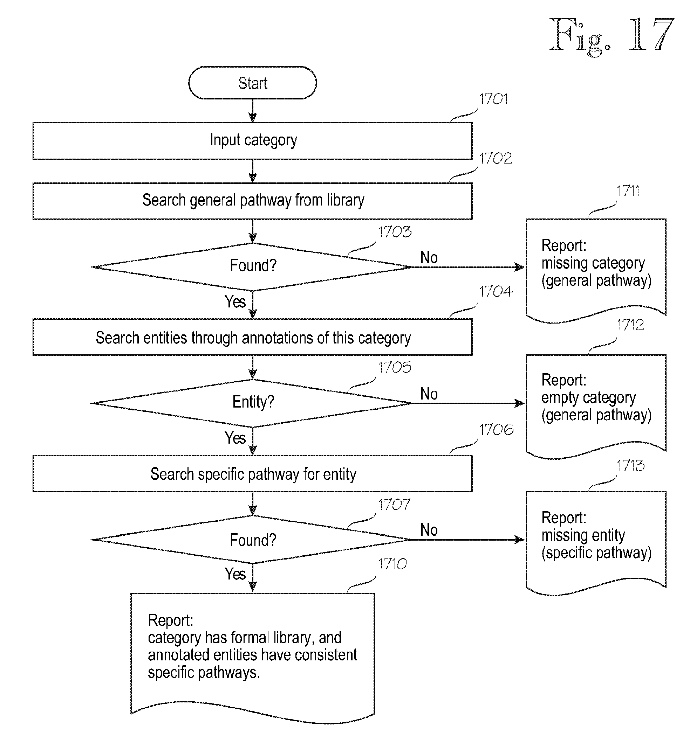

Figure 17 shows a flowchart of an embodiment of the consistency checker. The idea of a consistency checker is to automate the process of checking consistency between general pathway definitions 1002, specific pathway definitions 1004 and annotation sets 1006 described earlier, particularly in connection with Figures 10 - 13. The embodiment shown in Figure 17 checks the definition of a category.

In step 1701 the consistency checker receives an input which identifies category. In step 1702 the consistency checker searches a general pathway from the pathway library, based on the category identification. Step 1703 is a test to check if a matching general pathway is found. If not, the consistency checker proceeds to step 1711 for reporting a missing category. Otherwise the consistency checker searches through the stored entities for annotations of the category. The test in step 1705 checks if a matching entity is found. If not, the category is reported as empty in step 1712. In step 1706 the consistency checker searches for specific pathways which contain the entity found in step 1705. If none are found, the missing entity is reported in step 1713. Otherwise the consistency checker proceeds to step 1710 for reporting that the category has a formal library description and that the annotated entities have consistent specific pathways.

Figures 18A, 18B and 19 to 21 show how the invention can be used to formally express Gene Ontology (GO) definitions. The GO definitions for these figures are selected such that the figures contain very diverse material the description of which in a formal IMS system may not be trivial.

Figures 18A and 18B, which form a single logical drawing, illustrate modelling a Gene Ontology (GO) definition for molecular functions in general and certain exemplary molecular functions in particular. Reference numeral 1800 generally denotes a data structure for storing formal definitions for the term "molecular function". Reference numerals 1802A and 1802B denote, respectively, a GO identifier and a plaintext description for "molecular function". Reference numerals 1804A and 1804B denote, respectively, a GO identifier and a plaintext description for "catalytic activity", which is a subclass (sub-category) of "molecular function". Yet deeper into the definition hierarchy, reference numerals 1806A and 1806B denote, respectively, a GO identifier and a plaintext description for "adenylate cyclase activity", which is a subclass of "catalytic activity".

Reference numerals 1810A and 1810B denote, respectively, a GO identifier and a plaintext description for "transporter activity" which is another example of "molecular function". Reference numerals 1812A and 1812B denote a GO identifier and a plaintext description for "binding".

Finally, reference numerals 1814A and 1814B denote a GO identifier and a plaintext description for "toll binding". The definition for toll binding 1814B is interesting in that it is subclass of both transporter activity 1810B and binding 1812. This means that the definition for toll binding 1814B inherits features from two parents. This is possible because of the category binders 502 shown in Figure 5A. The explicit category binders 502 make it possible to bind an arbitrary numbers of parents to a category, as opposed to a rigid tree structure in which each category has only one parent (or none if the category is the root node of the tree).

One of the exemplary molecular functions shown in the data structure 1800 was "catalytic activity", denoted by reference numerals 1804A and 1804B. The GO definition for this function is: *"Catalysis of a biochemical reaction at physiological temperatures. In biologically catalysed reactions, the reactants are known as substrates, and the catalysts are naturally occurring macromolecular substances known as enzymes. Enzymes possess specific binding sites for substrates, and are usually composed wholly or largely of protein, but RNA that has catalytic activity (ribozyme) is often also regarded as enzymatic."*

The above-mentioned verbal definition can be easily stored in the IMS database for the benefit of human users, but its meaning is incomprehensible to current computers. Reference numeral 1820 denotes an equivalent pathway for providing a formal definition for the "catalytic activity" in terms of data structures 600 (see Figure 6). The pathway definition 1820 comprises an interaction "catalytic activity", denoted by reference numeral 1821. The pathway library comprises three connections related to the interaction 1821. There is a controller-type connection 1822 from category "catalytic activity", a substrate-type connection 1824 from category "substrate" and a product-type connection 1824 to category "product". The pathway identifier 1825 shows that the pathway definition is not limited to any specific location.

Another exemplary molecular function shown in the data structure 1800 was "adenylate cyclase activity", denoted by reference numerals 1806A and 1806B. The GO definition for this function is: *"Catalysis of the reaction: ATP = 3',5'-cyclic AMP + diphosphate".* Reference numeral 1830 denotes a pathway for providing a formal definition for the above definition for adenylate cyclase activity. In the pathway definition 1830, reference numeral 1831 denotes the interaction, reference numeral 1832 the controller function (in this example: catalysis), reference numeral 1833 denotes the substrate (ATP molecule), while reference numerals 1834 and 1835 denote the two products of the interaction, namely 3',5'-cyclic AMP and diphosphate.

Reference numeral 1840 denotes a pathway definition for the term "transporter activity". The pathway definition 1840 is analogous to the pathway definition 1820, and a detailed description is omitted.

Reference numeral 1850 denotes a pathway definition for the term "binding". Reference numeral 1851 denotes the interaction, reference numerals 1852 and 1853 denote the two substrate connections to the interaction 1851, while reference numeral 1854 denotes the product of the interaction.

Reference numeral 1860 denotes a pathway definition for the term "toll binding". The GO definition for this term is: *"Interacting selectively with the Toll protein, a transmembrane receptor".* In the pathway definition 1860, reference numeral 1861 denotes the interaction. The interaction 1861 has two substrate-type connections 1862 and 1864. The latter substrate-type connection leads from category "toll_binding" 1863, which also has a relation to location "transmembrane". It is worth noting that the category "toll_binding" 1863 has a dual role in the pathway 1860 because the category 1863 also has a controller-type connection 1865 to the interaction 1861. The interaction 1861 has two product-type connections. Reference numeral 1866 denotes a product-type connection to category "product", while reference numeral 1867 denotes the other product-type connection to category "bound receptor", which has a relation to location "transmembrane".

Figure 19 shows a formal definition for a process, namely "cell growth". The GO definition for "cell growth" is: *"The process by which a cell irreversibly increases in size over time by accretion and biosynthetic production of matter similar to that already present".* Reference numeral 1900 denotes an overall data structure which describes this definition. The data structure 1900 comprises a pathway definition 1910 and a set of state data (boundary conditions) 1920 and. Cell growth is a process in which a cell increases in size, but no biochemical entities are transformed to others. Hence, the pathway definition 1910 comprises an interaction 1911 which has no substrate or product connections. Instead, the interaction 1911 has a controller-type connection 1912 from category "cell growth" 1913 and an outcome-type connection 1914 to cell size 1915, which is expressed as VDL expression V[size]Cg[cell]. In plain text this means variable "size" of category "cell".

The set of state data 1920 for the pathway definition of "cell growth" comprises one boundary condition which states that variable 1921 (cell size at time T1) must be larger than variable 1922 (cell size at time T2) if variable 1923 (time T1) is larger than variable 1924 (time T4).

Figure 20 shows how the invention can be used to formally express a GO definition for "nucleus", which reads like this: *"A small, dense body one or more of which are present in the nucleus of eukaryotic cells. It is rich in RNA and protein, is not bounded by a limiting membrane, and is not seen during mitosis. Its prime function is the transcription of the nucleolar DNA into 45S ribosomal-precursor RNA, the processing of this RNA into 5.8S, 18S, and 28S components of ribosomal RNA, and the association of these components with 5S RNA and proteins synthesized outside the nucleolus. This association results in the formation of ribonucleoprotein precursors; these pass into the cytoplasm and mature into the 40S and 60S subunits of the ribosome".*

This definition reveals much about the nucleus but very little about the gene products which can be annotated to this category as "nucleus" gene products, if the GO guidelines are to be followed. While the above verbal definition describes several processes, they all take place outside the nucleus. Hence, a pathway definition 2000 for "nucleus" only contains a dummy product-type connection 2001 from an unspecified interaction to category "nucleus" 2002. This interpretation utilises a common part which applies to all biochemical entities annotated to this category. The common part is that all such biochemical entities are located in the nucleus. The pathway definition 2000 can be used to describe the structure and functionality of the nucleus itself. The simple pathway definition 2000 demonstrates the fact that the pathway definitions according to the invention tolerate pathways of which very little is known.

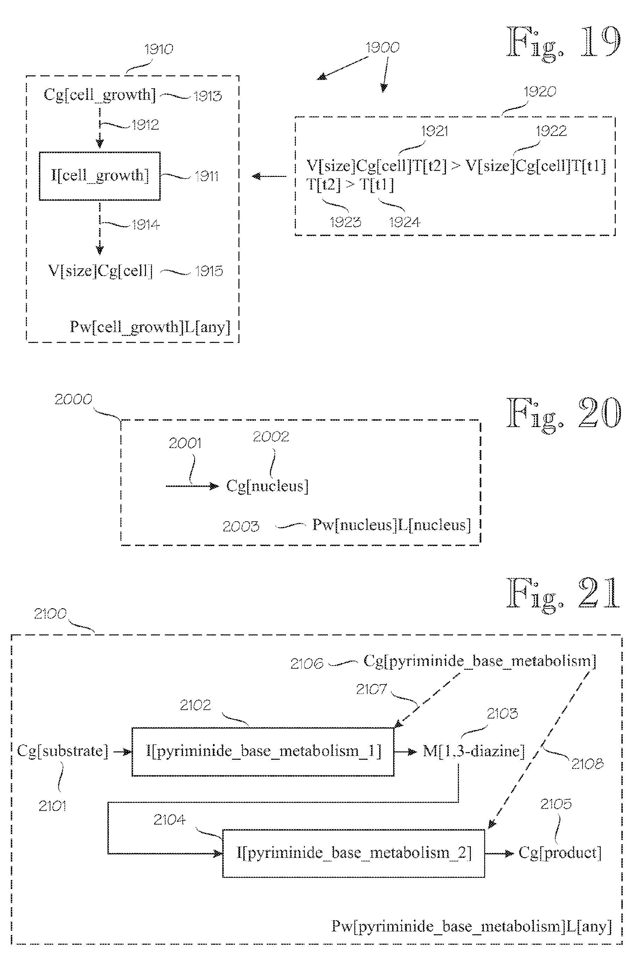

Finally, Figure 21 shows a pathway definition 2100 for pyriminide base metabolism, which means the conversion of a substrate 2101 to a product 2105 via 1,3-diazine 2103. The pathway definition 2100 is slightly more complex than the previous ones in that the pathway contains two instances of pyriminide base metabolism, denoted by reference numerals 2102 and 2104, of which the former produces 1,3-diazine from the substrate 2101 and the latter converts it into the product 2105. Reference numeral 2106 denotes the category for pyriminide base metabolism which has controller-type connections 2107, 2108 to the two instances 2102, 2104 of the interaction.

It will be apparent to a person skilled in the art that, as the technology advances, the inventive concept can be implemented in various ways. The invention and its embodiments are not limited to the examples described above but may vary within the scope of the claims.

**Acronyms**

Cg[x]: Category
GO: Gene Ontology
I[x]: Interaction
IMS: Information Management System
M[x]: Biochemical entity, eg molecule
VDL: Variable-Description Language

**Claims**

1. An electronic information management system for managing biochemical information, the information management system comprising data structures for modelling:

   - a plurality of biochemical entities (518, 616);
   - a hierarchical structure of a plurality of categories (508, 606, 722);
   - a plurality of pathways (602, 724);
   - a plurality of annotations (514), wherein each annotation associates a biochemical entity (518) to a category (508) and the plurality of annotations are collectively capable of forming a many-to-many relationship between the plurality of biochemical entities and the plurality of categories;

     - wherein the plurality of categories comprises a plurality of function categories, each function category describing a function of each biochemical entity associated to the function category;

   - a plurality of interactions (608);
   - a plurality of connections (604), wherein each connection:

     - associates a biochemical entity (616) or a category (606) to an interaction (608) and has a connection type (610), wherein the connection type is selected from a group which comprises substrate, product, controller and outcome, and
     - has a relation to a pathway (602) and each pathway has a relation to a biochemical location (624);

   - wherein the electronic information management system further comprises an interpretation logic (710) for interpreting each of several categories (722) as a pathway (724).

2. An information management system according to claim 1, further comprising a library of equivalent pathways of categories (720), wherein each equivalent pathway of a category comprises a set of connections which assigns the set of functions associated to the category to the biochemical entities associated to the category, and wherein the interpretation logic (710) is adapted to use the library of equivalent pathways of categories.

3. An information management system according to claim 1 or 2, further comprising an annotation logic (1000) for creating annotations (1006) based on the library (1002) and specific instances (1004) of pathways.

4. An information management system according to any one of the preceding claims, further comprising an instantiation logic (1100) for creating specific instances (1004) of pathways based on the library (1002), the annotations (1006) and an input set of biochemical entities.

5. An information management system according to any one of the preceding claims, further comprising a generalization logic for creating new categories (1002) and/or annotations (1006) and/or general pathways based on an input set of specific instances of pathways (1004).

6. An information management system according to any one of the preceding claims, further comprising a consistency checker for checking consistency between the annotations, the specific pathways and the library, based on specific instances of pathways and/or general pathways.

7. An information management system according to any one of the preceding claims, wherein at least one pathway comprises a hierarchical description of a biochemical entity (520, 618) and a hierarchical description of a location (40, 626).

8. An information management system according to any one of the preceding claims, further comprising:

   - means for storing description for the biochemical entities and locations in a variable description language (30), wherein the variable description language (30) comprises variable descriptions, each variable description comprising one or more pairs of keyword (32) and name (34) but no line terminator; and
   - a table (38) of permissible keywords.

9. A computer program product, executable in a computer system, the computer program product comprising program code portions for causing the computer system to implement the electronic information management system according to claim 1.

Fig. 1

CT

NW

Network

IMS

Server    S

DB processing, user interface...
Pathway processing (incl. categories)

Interpretation logic
Annotation logic
Instantation logic
Generalization logic
Consistency checker

DB

Database

Descriptions for
- biochemical entities
- categories
- pathways
- annotations
- interactions
- connections
Library of pathways of categories

Fig. 2

Component data (structure and other properties of components)

206

$V[quality\ attribute]M[x]T[t] = (value)$
$V[quantity\ attribute]M[x]T[t] = (value)$

I[4]

Outcome

I[1]    Product

M[x]    Substrate    I[2]

Controller

204    202

I[3]

204

System data (connections and kinetic laws)

State data (variables associated to components in a specific system in time and space)

# Fig. 3A

| 32 | | 33 | | 34 | | 35 | | 36 | | |
|---|---|---|---|---|---|---|---|---|---|---|
| Keyword | & | Opening delimiter | & | Name | & | Closing delimiter | & | Separator | & | 31 |

30

31

# Fig. 3B

| 38 | 38' | 38" |
|---|---|---|
| T | Time (relative) | T[-2.57E-3] |
| Ts | Time stamp | Ts[2002-11-26 14:00:00] |
| Sa | Sample | Sa[123456] |
| Po | Population | Po[2233445] |
| Id | Individual | Id[patient xyz] |
| En | Environment | Re[reagent a] |
| L | Location | L[yeast K2 mitocondria] |
| X/Y/Z | Spatial Z/Y/Z coordinate | X[0] relative to reference model |
| O | Organism | O[human] |
| V | Variable | V[concentration] |
| U | Unit | U[mol/l] |
| G | Gene | G[P53] |
| Tr | Transcript | Tr[mRNA from gene P53 to protein P53] |
| P | Protein | P[P53] |
| C | Compound | C[mannose] |
| M | Macromolecular complex | Mc[ribosome] |
| A | Abiotic stimuli | A[light] |
| I | Interaction | I[EC 1.2.3.4_P12345_F] |
| Cg | Category | Cg[Product] |

Fig. 4

40

Environment = ...

Population = ...

Individual = ...

Organism = ...

Organ = ...

Tissue = ...

Cellular compartment = ...

Molecule = ...

41

42 L[ABCD]

L[ABCD]X[0.25]Y[0.5]Z[0.75]

Fig. 5A

Fig. 5B

Fig. 6

Fig. 7

Fig. 8

Start

Input category — 802

Read definition — 804

Function
interpretation

806

Process
interpretation

Category type?

Location interpretation
- entity is located in
- entity is part of

808

810

812

Perform logic in Fig. 9A

Perform logic in Fig. 9B/9C

Perform logic in Fig. 9D

Produce connections of pathways — 814

Create library records — 816

End

Fig. 9A

```
                    ┌─────────────┐
                    │    Start    │
                    └──────┬──────┘
                           ▼
        ┌──────────────────────────────────┐
        │   Create a location (or "any")    │────── 902
        └──────────────────┬───────────────┘
                           ▼
        ┌──────────────────────────────────┐
        │  Create a pathway having that location │────── 904
        └──────────────────┬───────────────┘
                           ▼
        ┌──────────────────────────────────┐
        │  Create an interaction for the function │────── 906
        └──────────────────┬───────────────┘
```

908

Identify connection types of the biochemical
entities to be annotated to this category

| 911 | 912 | 913 | 914 |
|---|---|---|---|
| Substrate | Product | Outcome | Controller |

916

Create the relevant connection types to the pathway
between the category and the interaction

918

Yes ◄ More connections for this category ?

No

920

Identify other biochemical entities or categories connected to interactions
and identify connection types for them

| 921 | 922 | 923 | 924 |
|---|---|---|---|
| Substrate | Product | Outcome | Controller |

926

Create a connection of this type to the pathway
between the biochemical entity and the interaction

928

Yes ◄ More connections for other entities ?

No

```
                    ┌─────────────┐
                    │     End     │
                    └─────────────┘
```

**Fig. 9B**

```
                    ┌─────────────┐
                    │    Start    │
                    └──────┬──────┘
                           ▼
      ┌────────────────────────────────────────┐
      │    Create a location for the entity     │──── 941
      └────────────────────┬───────────────────┘
                           ▼
      ┌────────────────────────────────────────┐
      │   Create a pathway having that location │──── 942
      └────────────────────┬───────────────────┘
                           ▼
  ┌────────────────────────────────────────────────┐
  │     Identify connection types of the biochemical│──── 943
  │     entities to be annotated to this category   │
  └──────────────────────┬─────────────────────────┘
                         ▼
            ┌────────────────────┐
            │      Product       │──── 944
            └─────────┬──────────┘
                      ▼
  ┌────────────────────────────────────────────────┐
  │     Create a dummy connection to pathway        │──── 945
  │  between category and an unspecified interaction │
  └──────────────────────┬─────────────────────────┘
                         ▼
                ┌─────────────┐
                │     End     │
                └─────────────┘
```

Fig. 9C

```
                          ┌─────────┐
                          │  Start  │
                          └─────────┘
                               │
                               ▼
        ┌──────────────────────────────────────────────┐
        │        Create a location (or "any")          │──── 951
        └──────────────────────────────────────────────┘
                               │
                               ▼
        ┌──────────────────────────────────────────────┐
        │      Create a pathway having that location    │──── 952
        └──────────────────────────────────────────────┘
                               │
                               ▼
        ┌──────────────────────────────────────────────┐
        │       Create an interaction for the function  │──── 953
        └──────────────────────────────────────────────┘
                               │
                               ▼
    ┌──────────────────────────────────────────────────────┐
    │     Identify connection types of the biochemical     │──── 954
    │     entities to be annotated to this category        │
    └──────────────────────────────────────────────────────┘
                │
                ▼
        ┌──────────────┐
        │  Substrate   │──── 955
        └──────────────┘
                │
                ▼
    ┌──────────────────────────────────────────────────────┐
    │        Create the relevant connection types to       │──── 956
    │        pathway between category and interaction       │
    └──────────────────────────────────────────────────────┘
                               │
                               ▼
    ┌──────────────────────────────────────────────────────┐
    │ Identify other biochemical entities or categories     │──── 957
    │ connected to interactions and identify connection     │
    │ types for them                                        │
    └──────────────────────────────────────────────────────┘
         │          │            │            │
         ▼          ▼            ▼            ▼
    ┌─────────┐ ┌─────────┐ ┌─────────┐ ┌──────────┐
    │Substrate│ │ Product │ │ Outcome │ │Controller│
    └─────────┘ └─────────┘ └─────────┘ └──────────┘
       961         962         963         964
         │          │            │            │
         ▼          ▼            ▼            ▼
    ┌──────────────────────────────────────────────────────┐
    │    Create a connection of this type to the pathway    │──── 966
    │    between the biochemical entity and the interaction  │
    └──────────────────────────────────────────────────────┘
                               │
                               ▼
                        ◇ More              968
              Yes  ◇ connections for other ◇
           ◄──────◇     entities ?        ◇
                        ◇                  ◇
                               │ No
                               ▼
                          ┌─────────┐
                          │   End   │
                          └─────────┘
```

Fig. 9D

```
                    ┌──────────────┐
                    │    Start     │
                    └──────┬───────┘
                           ▼
      ┌─────────────────────────────────────────┐
      │        Create a location (or "any")      │── 971
      └─────────────────────┬───────────────────┘
                            ▼
      ┌─────────────────────────────────────────┐
      │ Create a pathway having a relation to the location │── 972
      └─────────────────────┬───────────────────┘
                            ▼
      ┌─────────────────────────────────────────┐
      │   Create interactions for functions of the process │── 973
      └─────────────────────┬───────────────────┘
                            ▼
```

Identify connection types of the biochemical entities to be annotated to this category — 974

| Substrate | Product | Outcome | Controller |
|-----------|---------|---------|------------|

975    976    977    978

Create the relevant connection types to pathway between category and interaction — 979

More connections for this category? — 981
Yes / No

Identify other biochemical entities or categories connected to interactions and identify connection types for them — 982

| Substrate | Product | Outcome | Controller |
|-----------|---------|---------|------------|

983    984    985    986

Create a connection of this type to the pathway between the biochemical entity and the interaction — 988

More connections for other entities? — 989
Yes / No

Identify potential state_data_condition for initial, end or boundary conditions — 991

Create relevant state_data_condition related to the pathway — 992

End

Fig. 10

Fig. 11

Fig. 12

Fig. 13

1002

Cg[C]

M[x4] → I[x] → M[x5]

1004

M[x2]

M[x4] → I[y] → M[x5]

M[x3]

M[x4] → I[z] → M[x5]

1006

M[x2]
        → Cg[C]
M[x3]

1300

1302

Report of potential inconsistencies

Fig. 14

Start

Input category and specific pathways — 1401

Use the category to retrieve a general pathway representing the category — 1402

Use the general pathway as network pattern where category is used as wildcard to find relevant connections from specific pathway — 1403

Identify specific biochemical entities in the specific connections matched from the specific pathways, the biochemical entities able to replace the category — 1404

Create an annotation for each identified biochemical entity to category — 1405

End

Fig. 15

```
                          ┌──────────┐
                          │  Start   │
                          └────┬─────┘
                               ▼
┌──────────────────────────────────────────────────────────────┐
│ Input: biochemical entities and pathway (for new specific     │ ─── 1501
│ instances)                                                     │
└──────────────────────────────┬───────────────────────────────┘
                               ▼
                                                    1502
              ◇───────────────────────────────◇      Yes          ┌──────────┐
              │   All input entities processed? │ ───────────────▶│   End    │
              ◇───────────────────────────────◇                   └──────────┘
                               │ No
                               ▼
┌──────────────────────────────────────────────────────────────┐
│ Get annotations of biochemical entity and related categories  │ ─── 1503
└──────────────────────────────┬───────────────────────────────┘
         1505                   ▼                    1504
  ┌───────────┐   No   ◇──────────────────────────────◇
  │  Next     │◀───────│   More categorie to process?  │
  │  entity   │        ◇──────────────────────────────◇
  └───────────┘                 │ Yes
                               ▼
┌──────────────────────────────────────────────────────────────┐
│ Use the category to obtain a general pathway representing the  │ ─── 1506
│ category                                                       │
└──────────────────────────────┬───────────────────────────────┘
                               ▼
┌──────────────────────────────────────────────────────────────┐
│ Get the connections of the general pathway from the database   │ ─── 1507
│ to a buffer                                                    │
└──────────────────────────────┬───────────────────────────────┘
                               ▼
┌──────────────────────────────────────────────────────────────┐
│ Change the pathway relation of the connections                 │ ─── 1508
│ in the buffer to point to a new specific pathway               │
└──────────────────────────────┬───────────────────────────────┘
                               ▼
┌──────────────────────────────────────────────────────────────┐
│ Replace category related from connections in the buffer        │ ─── 1509
│ by a biochemical entity annotated to the category              │
└──────────────────────────────┬───────────────────────────────┘
                               ▼
┌──────────────────────────────────────────────────────────────┐
│ Store modified connections in the buffer into the database     │ ─── 1510
│ for a new specific pathway                                     │
└──────────────────────────────┬───────────────────────────────┘
                               ▼
┌──────────────────────────────────────────────────────────────┐
│ Next category                                                  │ ─── 1511
└──────────────────────────────────────────────────────────────┘
```

Fig. 16

```
                    ┌─────────────────┐
                    │      Start       │
                    └─────────────────┘
                             │
                             ▼
    ┌──────────────────────────────────────────────────────┐
    │              Input specific pathway                    │────── 1601
    └──────────────────────────────────────────────────────┘
                             │
                             ▼
    ┌──────────────────────────────────────────────────────┐
    │ Create reduced pathway from specific pathway by        │
    │ removing connections that match connections of         │────── 1602
    │ existing general pathways for existing categories      │
    └──────────────────────────────────────────────────────┘
                             │
                             ▼
    ┌──────────────────────────────────────────────────────┐
    │    Index connections of the reduced pathway for        │────── 1603
    │                  iteration                             │
    └──────────────────────────────────────────────────────┘
                             │
                             ▼
```

1604

No ◄──── More connections to process?

Yes

Compare selected connection with all other connections on list concerning type and relation to entity but not concerning other fields and create similarity data elements for connecting similar connections (connectionA, interactionA, type, connectionZ, interactionZ) ────── 1605

Delete the current connection from the list ────── 1606

Create a new functional category for the different entities having a controller type connection to interactions whose similarity meets a predetermined criterion. ────── 1607

Full similarity: similar substrates are converted to similar products
Partial similarity: some difference in substrates and/or products tolerated

```
                    ┌─────────────────┐
                    │       End        │
                    └─────────────────┘
```

Fig. 17

```
                    ┌──────────────┐
                    │    Start     │
                    └──────┬───────┘
                           │                          1701
                    ┌──────▼───────────────────────┐
                    │      Input category          │
                    └──────┬───────────────────────┘
                           │                          1702
                    ┌──────▼───────────────────────┐
                    │ Search general pathway from library │
                    └──────┬───────────────────────┘
                           │              1703
                     ◇─────▼──────◇   No                    1711
                    ◇   Found?    ◇──────────►  ┌──────────────────┐
                     ◇───────────◇              │ Report:          │
                           │ Yes       1704     │ missing category │
                    ┌──────▼───────────────────────┐ │ (general pathway)│
                    │ Search entities through annotations of this category │
                    └──────┬───────────────────────┘              1712
                           │              1705
                     ◇─────▼──────◇   No                    ┌──────────────────┐
                    ◇   Entity?   ◇──────────►              │ Report:          │
                     ◇───────────◇                          │ empty category   │
                           │ Yes       1706                 │ (general pathway)│
                    ┌──────▼───────────────────────┐              1713
                    │ Search specific pathway for entity │
                    └──────┬───────────────────────┘
                           │              1707
                     ◇─────▼──────◇   No                    ┌──────────────────┐
                    ◇   Found?    ◇──────────►              │ Report:          │
                     ◇───────────◇                          │ missing entity   │
                           │ Yes       1710                 │ (specific pathway)│
                    ┌──────▼───────────────────────┐
                    │ Report:                        │
                    │ category has formal library, and │
                    │ annotated entities have consistent │
                    │ specific pathways.             │
                    └────────────────────────────────┘
```

1800

Fig. 18A

1802A    1802B

| GO:0003674: | molecular_function |

GO:0003824: | catalytic_activity ←— 1804A, 1804B

GO:0004016: | adenylate_cyclase_activity ←— 1806A, 1806B

...

GO:0005215: | transporter_activity ←— 1810A, 1810B

GO:0005488: | binding ←— 1812A, 1812B

1814A, 1814B

GO:0005121: | toll_binding ←—

Cg[catalytic_activity]

1822

1823                          1824

Cg[substrate] —→ I[catalytic_activity] —→ Cg[product]          1820

1821

1825 —— Pw[catalytic_activity]L[any]

Cg[adenylate_cyclase_activity]

1832                          1834

M[3'_5'-cyclic_AMP]

1833

M[ATP] —→ I[adenylate_cyclase_activity]                        1830

M[diphosphate]

1831                          1835

1836 —— Pw[adenylate_cyclase_activity]L[any]

Fig. 18B

1840

Cg[transporter_activity]L[any]

1843          ⌐ 1842     1844

Cg[substrate]L[any_2] ——→  I[transporter_activity]  ——→ Cg[product]L[any_3]

1841

1845 ⌐ Pw[transporter_activity]L[any]

1850

1852                          1854

Cg[binding] ↘                         ↗
              I[binding]  ——→ Cg[product]
Cg[substrate] ↗

1853     1851

1855 ⌐ Pw[binding]L[any]

1860

Cg[toll_binding]L[transmembrane] ⌐ 1863

1864 ⌐        ⌐ 1865

1862                    1866

Cg[substrate]L[any_1] ——→  I[toll_binding]  ——→ Cg[product]L[any_2]

1867

1861        Cg[bound_receptor]L[transmembrane]

1868 ⌐ Pw[toll_binding]L[transmembrane]

30

**Fig. 19**

1910

Cg[cell_growth] ～ 1913

↓ ～ 1912

| I[cell_growth] | ～ 1911

↓ ～ 1914

V[size]Cg[cell] ～ 1915

Pw[cell_growth]L[any]

1900

1920

V[size]Cg[cell]T[t2] > V[size]Cg[cell]T[t1]  1921  1922
T[t2] > T[t1]

1923  1924

**Fig. 20**

2000

2001  2002

→ Cg[nucleus]

2003 ～ Pw[nucleus]L[nucleus]

**Fig. 21**

2100

2106 ～ Cg[pyriminide_base_metabolism]

2107

2102

Cg[substrate] → | I[pyriminide_base_metabolism_1] | → M[1,3-diazine]  2103

2101

2108

2104

→ | I[pyriminide_base_metabolism_2] | → Cg[product]  2105

Pw[pyriminide_base_metabolism]L[any]

# EP 1 770 571 A1

**European Patent Office**

## EUROPEAN SEARCH REPORT

**Application Number**

EP 06 12 1164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 2005/003999 A1 (MEDICEL OY [FI]; VARPELA PERTTELI [FI]; KOLMER MEELIS [FI]; PELLIKKA T) 13 January 2005 (2005-01-13)<br>* the whole document *<br>----- | 1-9 | INV.<br>G06F19/00 |

**TECHNICAL FIELDS SEARCHED (IPC)**

G06F

The present search report has been drawn up for all claims

| Place of search | Date of completion of the search | Examiner |
|---|---|---|
| The Hague | 17 October 2006 | Swarén, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

32

## ANNEX TO THE EUROPEAN SEARCH REPORT
## ON EUROPEAN PATENT APPLICATION NO.

EP 06 12 1164

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2006

| Patent document cited in search report | Publication date | Patent family member(s) | Publication date |
|---|---|---|---|
| WO 2005003999 A1 | 13-01-2005 | AU 2004254729 A1<br>CA 2531131 A1 | 13-01-2005<br>13-01-2005 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005003999 A
- EP 1494159 A
- US 10883648 B